(19) **Europäisches Patentamt**
**European Patent Office**
**Office européen des brevets**

(11) **EP 4 625 382 A1**

(12) **EUROPEAN PATENT APPLICATION**
published in accordance with Art. 153(4) EPC

(43) Date of publication:
**01.10.2025 Bulletin 2025/40**

(21) Application number: **23899922.1**

(22) Date of filing: **04.12.2023**

(51) International Patent Classification (IPC):
***G08B 21/24*** *(2006.01)*

(52) Cooperative Patent Classification (CPC):
**G01J 1/42; G01J 5/00; G02C 7/10; G08B 21/18; G08B 21/24**

(86) International application number:
**PCT/CN2023/136187**

(87) International publication number:
**WO 2024/120345 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.12.2022 CN 202211556409**

(71) Applicant: **Huawei Technologies Co., Ltd.**
**Shenzhen, Guangdong 518129 (CN)**

(72) Inventors:
- **ZHAO, Shuai**
  **Shenzhen, Guangdong 518129 (CN)**
- **REN, Huichao**
  **Shenzhen, Guangdong 518129 (CN)**
- **CAO, Yu**
  **Shenzhen, Guangdong 518129 (CN)**
- **HOU, Zhongjie**
  **Shenzhen, Guangdong 518129 (CN)**

(74) Representative: **Maiwald GmbH**
**Engineering**
**Elisenhof**
**Elisenstrasse 3**
**80335 München (DE)**

(54) **PROTECTION PROMPTING METHOD AND ELECTRONIC DEVICE**

(57) Embodiments of the present invention provide a protection prompt method and an electronic device. In the technical solutions provided in embodiments of the present invention, the electronic device includes a first sensor, a second sensor, and a third sensor. The electronic device obtains an ultraviolet light intensity captured by the first sensor, a first temperature captured by the second sensor, and a second temperature captured by the third sensor; obtains an ultraviolet radiation amount based on the ultraviolet light intensity; obtains a core body temperature of a user based on the ultraviolet light intensity, the first temperature, and the second temperature; and provides a protection prompt for the user based on the ultraviolet radiation amount and the core body temperature. This provides a continuous monitoring capability and accurately detects the core body temperature of the human body, thereby providing an effective protection reminder for the user.

FIG. 13

**Description**

This application claims priority to Chinese Patent Application No. 202211556409.2, filed with the China National Intellectual Property Administration on December 6, 2022 and entitled "PROTECTION PROMPT METHOD AND ELECTRONIC DEVICE", which is incorporated herein by reference in its entirety.

**TECHNICAL FIELD**

The present invention relates to the field of computer technologies, and in particular, to a protection prompt method and an electronic device.

**BACKGROUND**

An intensity and time of ultraviolet light received by a human body are key parameters for skin health. In addition, ultraviolet radiation causes a rapid increase in a skin surface temperature of the human body, but a core temperature of the human body increases slightly. In a current ultraviolet detection method, a continuous monitoring capability is not provided, and the core body temperature of the human body cannot be accurately detected. Consequently, an effective protection reminder cannot be provided for a user.

**SUMMARY**

In view of this, embodiments of the present invention provide a protection prompt method and an electronic device, to provide a continuous monitoring capability and accurately detect a core body temperature of a human body, thereby providing an effective protection reminder for a user.

According to a first aspect, an embodiment of the present invention provides a protection prompt method, applied to an electronic device. The electronic device includes a first sensor, a second sensor, and a third sensor, and the method includes:

obtaining an ultraviolet light intensity captured by the first sensor, a first temperature captured by the second sensor, and a second temperature captured by the third sensor;
obtaining an ultraviolet radiation amount based on the ultraviolet light intensity;
obtaining a core body temperature of a user based on the ultraviolet light intensity, the first temperature, and the second temperature; and
providing a protection prompt for the user based on the ultraviolet radiation amount and the core body temperature. In embodiments of the present invention, in an ultraviolet radiation scenario, the ultraviolet radiation amount and the core body temperature that are received by the user can be continuously monitored, and an effective protection reminder is provided for the user based on the ultraviolet radiation amount and the core body temperature.

With reference to the first aspect, in some implementations of the first aspect, the obtaining the ultraviolet radiation amount based on the ultraviolet light intensity includes:
performing cumulative summation on the ultraviolet light intensity to obtain the ultraviolet radiation amount. During actual monitoring, the ultraviolet sensor is invoked once at an interval to obtain the ultraviolet light intensity. Therefore, the ultraviolet radiation amount calculated in the cumulative summation manner based on the ultraviolet light intensity and the interval time is accurate.

With reference to the first aspect, in some implementations of the first aspect, the obtaining the core body temperature of the user based on the ultraviolet light intensity, the first temperature, and the second temperature includes:

if the ultraviolet light intensity is less than a first threshold, obtaining the core body temperature of the user based on the first temperature and the second temperature; or
if the ultraviolet light intensity is greater than or equal to the first threshold, obtaining the core body temperature of the user based on the ultraviolet light intensity, the first temperature, and the second temperature. In embodiments of the present invention, a current ultraviolet radiation degree is determined based on the ultraviolet light intensity. When the ultraviolet light intensity is less than the first threshold, it indicates that the user is in a low ultraviolet radiation scenario, and the ultraviolet light has small impact on the core body temperature of the user. Therefore, when the core body temperature is calculated, the ultraviolet light intensity may not be considered, and only an ambient temperature and a skin temperature of the user are considered. When the ultraviolet light intensity is greater than or equal to the first threshold, it indicates that the user is in a scenario with high ultraviolet radiation, and the ultraviolet light has large impact on the core body temperature of the user. Therefore, the ultraviolet light intensity needs to be considered when the core body temperature is calculated. In this way, the ultraviolet light intensity is compared with the first threshold, and core body temperature detection models are separately established based on scenarios with radiation intensities, so that accuracy of core body temperature detection can be effectively improved. A method for calculating the core body temperature in scenarios with different ultraviolet radiation is implemented.

With reference to the first aspect, in some implementations of the first aspect, the obtaining the core body temperature of the user based on the first temperature and the second temperature includes:
calculating the first temperature and the second temperature by using a multiple linear regression model, to obtain the core body temperature.

With reference to the first aspect, in some implementations of the first aspect, the obtaining the core body temperature of the user based on the ultraviolet light intensity, the first temperature, and the second temperature includes:
calculating the ultraviolet light intensity, the first temperature, and the second temperature by using an XGBoost model, to obtain the core body temperature. When the human body is exposed to strong ultraviolet radiation, both the skin temperature of the human head and the ambient temperature of the environment in which the smart glasses are located increase greatly. However, due to a body temperature adjustment mechanism of the human body, the core body temperature increases slightly. If only the skin temperature and the ambient temperature are used to calculate the core body temperature without considering the ultraviolet radiation intensity, a large deviation may occur, resulting in an incorrect body temperature exception reminder. In the case of strong ultraviolet radiation, the coupling introduced by the ultraviolet light intensity can be added based on the skin temperature and the ambient temperature to accurately calculate the core temperature and provide an accurate result for the user.

With reference to the first aspect, in some implementations of the first aspect, the providing an ultraviolet protection prompt and/or a temperature protection prompt for the user based on the ultraviolet radiation amount and the core body temperature includes:

providing the ultraviolet protection prompt for the user based on the ultraviolet radiation amount; and
providing the temperature protection prompt for the user based on the core body temperature. In embodiments of the present invention, the ultraviolet protection prompt and the temperature protection prompt are separately provided for the user based on the ultraviolet radiation amount and the core body temperature. This is applicable to scenarios of ultraviolet radiation monitoring and core body temperature monitoring. The electronic device is used to monitor ultraviolet radiation and a body temperature change of the user, and remind the user in time to perform protection in time, to protect skin health of the user and avoid a risk such as heatstroke.

With reference to the first aspect, in some implementations of the first aspect, providing the ultraviolet protection prompt for the user based on the ultraviolet radiation amount includes:

if the ultraviolet radiation amount is less than or equal to a second threshold, skipping providing the ultraviolet protection prompt; or
if the ultraviolet radiation amount is greater than the second threshold, providing the ultraviolet protection prompt for the user based on the ultraviolet radiation amount. In embodiments of the present invention, if the ultraviolet radiation amount is less than or equal to the second threshold, it indicates that the user receives a small ultraviolet radiation amount, and no ultraviolet protection prompt is provided. If the ultraviolet radiation amount is greater than the second threshold, it indicates that the user receives a large ultraviolet radiation amount, and the ultraviolet protection prompt is provided for the user based on the ultraviolet radiation amount.

With reference to the first aspect, in some implementations of the first aspect, providing the temperature protection prompt for the user based on the core body temperature includes:

if the core body temperature is greater than or equal to a third threshold and less than or equal to a fourth threshold, skipping providing the temperature protection prompt;
if the core body temperature is less than the third threshold, providing a prompt of paying attention to hypothermia protection for the user based on the core body temperature; or
if the core temperature is greater than the fourth threshold, providing a prompt of paying attention to heatstroke prevention for the user based on the core body temperature. In embodiments of the present invention, if the core body temperature is greater than or equal to the third threshold and less than or equal to the fourth threshold, it indicates that the core body temperature of the user is within a normal body temperature range, and no temperature protection prompt is provided. If the core body temperature is less than the third threshold, it indicates that the core body temperature of the user is already less than the lowest normal body temperature, the prompt of paying attention to hypothermia protection is provided for the user based on the core body temperature. If the core temperature is greater than the fourth threshold, it indicates that the core body temperature of the user is greater than the highest normal body temperature, the prompt of paying attention to heatstroke prevention is provided for the user based on the core body temperature.

With reference to the first aspect, in some implementations of the first aspect, the second threshold is a personalized threshold corresponding to a physiological parameter of the user. Embodiments of the present invention provide a capability of setting the second threshold in a personalized manner, to meet a requirement of a wider use group.

With reference to the first aspect, in some implementations of the first aspect, before the obtaining the ultraviolet light intensity captured by the first sensor, the first temperature captured by the second sensor, and the second temperature captured by the third sensor, the method further includes:

obtaining the second threshold in response to a change operation performed by the user on a target control used to display the second threshold. In embodiments of the present invention, the second threshold is set by the user. For example, a user who works outdoors with high ultraviolet radiation for a long time may adjust the second threshold based on a requirement of the user.

With reference to the first aspect, in some implementations of the first aspect, the first temperature is an ambient temperature, and the second temperature is a skin temperature of the user.

With reference to the first aspect, in some implementations of the first aspect, the electronic device includes smart glasses. Embodiments of the present invention are applicable to scenarios of ultraviolet radiation monitoring and core body temperature monitoring. A wearable device is used to monitor ultraviolet radiation and a body temperature change of the user, and remind the user in time to perform protection, to protect skin health of the user and avoid a risk such as heatstroke. The smart glasses are worn on the face of the user, so that an ultraviolet radiation amount received by the face of the user can be more accurately monitored.

With reference to the first aspect, in some implementations of the first aspect, a manner of providing the ultraviolet protection prompt and/or the temperature protection prompt for the user includes one or any combination of the following options: automatically changing a color of a lens, displaying prompt content on the lens with a display function, playing the prompt content through a speaker, and providing the ultraviolet protection prompt and/or the temperature protection prompt through another electronic device. Embodiments of the present invention provide multiple protection prompt manners to meet various requirements of different users.

With reference to the first aspect, in some implementations of the first aspect, the smart glasses include an electrochromic lens.

With reference to the first aspect, in some implementations of the first aspect, the automatically changing a color of the lens includes:

adjusting an applied voltage of an electrochromic material in the lens based on the ultraviolet light intensity. In embodiments of the present invention, the color of the lens is automatically adjusted, so that the user can be directly and intuitively prompted, and an ultraviolet radiation amount received by the eyes of the user can be reduced, thereby protecting the eyes.

With reference to the first aspect, in some implementations of the first aspect, the obtaining the ultraviolet light intensity captured by the first sensor includes:

obtaining the ultraviolet light intensity captured by the first sensor; and
adjusting an invocation frequency of the first sensor based on the ultraviolet light intensity. The user pays more attention to an amount of received strong ultraviolet radiation (for example, $U_{Vi} > 5$). Therefore, the invocation frequency of the first sensor may be dynamically adjusted based on the ultraviolet light intensity detected by the first sensor, so that power consumption of the device can be effectively reduced, a battery life of the wearable device can be increased, and continuous low-power monitoring for a long time can be implemented.

With reference to the first aspect, in some implementations of the first aspect, the first sensor and the second sensor are located in positions that are in a temple of the smart glasses and that are close to a front panel. The first sensor and the second sensor are located in the temple of the smart glasses, so that a hardware layout of the smart glasses is simpler, and an FPC flexible flat cable passing through a hinge does not need to be used, so that hardware overheads can be reduced. In addition, although the ultraviolet light intensity received by the first sensor is slightly different from an ultraviolet light intensity on the front face, the ultraviolet light intensity may also be corrected according to an algorithm.

With reference to the first aspect, in some implementations of the first aspect, the first sensor and the second sensor are located on a front panel of a lens frame of the smart glasses. When the user wears the smart glasses, because the first sensor is located in front of the face, an ultraviolet light intensity received by the first sensor is almost consistent with an ultraviolet light intensity received by the face. Therefore, an ultraviolet radiation amount received by the face can be accurately evaluated, and a monitoring result is more accurate.

With reference to the first aspect, in some implementations of the first aspect, the first sensor and the second sensor are located in positions that are close to a temple and that are in the front panel of the lens frame. When the user wears the smart glasses, because the first sensor is located in front of the face, an ultraviolet light intensity received by the first sensor is almost consistent with the ultraviolet light intensity received by the face. Therefore, an ultraviolet radiation amount received by the face can be accurately evaluated, and a monitoring result is more accurate.

With reference to the first aspect, in some implementations of the first aspect, the first sensor and the second sensor are located in middle positions of the front panel of the lens frame. When the user wears the smart glasses, because the first sensor is located right in front of the face, an ultraviolet light intensity received by the first sensor is completely consistent with an ultraviolet light intensity received by the face. Therefore, an ultraviolet radiation amount received by the face can be more accurately evaluated, and a monitoring result is more accurate.

With reference to the first aspect, in some implementations of the first aspect, the second sensor is located outside the smart glasses, and the third sensor is located inside the temple of the smart glasses. The second sensor is located outside the smart glasses, and is configured to capture an ambient temperature. The third sensor is located inside the temple of the smart glasses, and is configured to capture a skin temperature of the user.

With reference to the first aspect, in some implementations of the first aspect, the electronic device includes a smart watch or a smart headset.

According to a second aspect, an embodiment of the present invention provides an electronic device, including a processor and a memory. The memory is configured to store a computer program, the computer program includes program instructions, and when the processor runs the program instructions, the electronic device is enabled to perform the steps of the foregoing methods.

According to a third aspect, an embodiment of the present invention provides a computer-readable storage medium. The computer-readable storage medium stores a computer program, the computer program includes program instructions, and when the program requests to be run by a computer, the computer is enabled to perform the foregoing methods.

According to a fourth aspect, an embodiment of the present invention provides a computer program product. The computer program product includes instructions, and when the computer program product runs on a computer or any at least one processor, the computer is enabled to perform the functions/steps in the foregoing methods.

## BRIEF DESCRIPTION OF DRAWINGS

FIG. 1 is a diagram of a structure of an electronic device according to an embodiment of the present invention;
FIG. 2 is a block diagram of a software structure of an electronic device 100 according to an embodiment of the present invention;
FIG. 3 is a diagram of an electronic device according to an embodiment of the present invention;
FIG. 4A to FIG. 4D are a diagram of generating a personalized threshold by a mobile phone based on a physiological parameter of a user according to an embodiment of the present invention;
FIG. 5 is a diagram in which a color of a lens becomes deeper as an applied voltage increases according to an embodiment of the present invention;
FIG. 6 is a diagram of displaying prompt content by a lens according to an embodiment of the present invention;
FIG. 7 is a diagram of displaying prompt content by a smartwatch according to an embodiment of the present invention;
FIG. 8 is another diagram of displaying prompt content by a smartwatch according to an embodiment of the present invention;
FIG. 9 is a diagram of displaying an ultraviolet radiation monitoring interface by a mobile phone according to an embodiment of the present invention;
FIG. 10 is a diagram of an ultraviolet radiation monitoring interface after a user performs a slide-up operation in FIG. 9;
FIG. 11 is a diagram of another electronic device according to an embodiment of the present invention;
FIG. 12 is a diagram of another electronic device according to an embodiment of the present invention;
FIG. 13 is a flowchart of a protection prompt method according to an embodiment of the present invention;
FIG. 14 is a specific flowchart of obtaining an ultraviolet radiation amount based on an ultraviolet light intensity in FIG. 13;
FIG. 15 is a specific flowchart of obtaining a core body temperature of a user based on an ultraviolet light intensity, a first temperature, and a second temperature in FIG. 13;
FIG. 16 is a specific flowchart of providing a protection prompt for a user based on an ultraviolet radiation amount and a core body temperature in FIG. 13;
FIG. 17 is a specific flowchart of providing an ultraviolet protection prompt for a user based on the ultraviolet radiation amount in FIG. 16;
FIG. 18 is a specific flowchart of providing a temperature protection prompt for a user based on the core body temperature in FIG. 16;

FIG. 19 is a flowchart of automatically changing a color of a lens according to an embodiment of the present invention;

FIG. 20 is a flowchart of displaying prompt content on a lens with a display function according to an embodiment of the present invention;

FIG. 21 is a flowchart of playing prompt content through a speaker according to an embodiment of the present invention; and

FIG. 22 is a diagram of a structure of an electronic device according to an embodiment of the present invention.

## DESCRIPTION OF EMBODIMENTS

To make technical solutions in the present invention more comprehensible, the following describes embodiments of the present invention in detail with reference to accompanying drawings.

It should be clear that the described embodiments are merely some but not all of embodiments of the present invention. All other embodiments obtained by a person of ordinary skill in the art based on embodiments of the present invention without creative efforts shall fall within the protection scope of the present invention.

The terms used in embodiments of the present invention are merely for the purpose of illustrating specific embodiments, and are not intended to limit the present invention. Terms "a", "the foregoing", and "the" of singular forms used in embodiments and the appended claims of the present invention are also intended to include plural forms, unless otherwise specified in the context clearly.

It should be understood that a term "and/or" used in this specification describes only an association relationship between associated objects and represents that three relationships may exist. For example, A and/or B may represent the following three cases: Only A exists, both A and B exist, and only B exists. In addition, the character "/" in this specification usually indicates an "or" relationship between the associated objects.

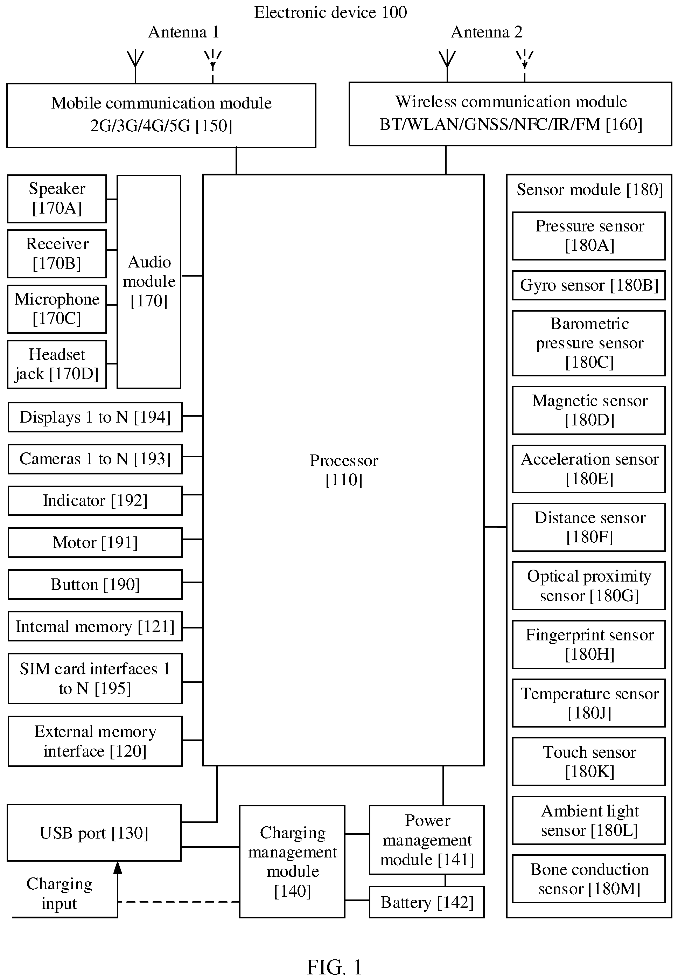

FIG. 1 is a diagram of a structure of an electronic device 100.

The electronic device 100 may include a processor 110, an external memory interface 120, an internal memory 121, a universal serial bus (universal serial bus, USB) port 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100. In some other embodiments of this application, the electronic device 100 may include more or fewer components than those shown in the figure, or a combination of a part of the components, or splits from a part of the components, or an arrangement of different components. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent devices, or may be integrated into one or more processors.

The controller may generate an operation control signal based on an instruction operation code and a time sequence signal, to complete control of instruction fetching and instruction execution.

A memory may be further disposed in the processor 110, and is configured to store instructions and data. In some embodiments, the memory in the processor 110 is a cache memory. The memory may store instructions or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/-transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) port, and/or the like.

The I2C interface is a two-way synchronization serial bus, and includes a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be configured to perform audio communication, and sample, quantize, and encode an analog signal. In some embodiments, the audio module 170 may be coupled to the wireless communication module 160 through a PCM bus interface. In some embodiments, the audio module 170 may also transmit an audio signal to the wireless communication module 160 through the PCM interface, to implement a function of answering a call through a Bluetooth headset. Both the I2S interface and the PCM interface may be configured to perform audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral device, for example, the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device 100. The processor 110 communicates with the display 194 through the DSI interface, to implement a display function of the electronic device 100.

The GPIO interface may be configured by using software. The GPIO interface may be configured as a control signal, or may be configured as a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB port 130 is a port that conforms to a USB standard specification, and may be specifically a mini USB port, a micro USB port, a USB type-C interface, or the like. The USB port 130 may be configured to connect to a charger to charge the electronic device 100, or may be configured to transmit data between the electronic device 100 and a peripheral device, or may be configured to connect to a headset for playing audio through the headset. Alternatively, the port may be configured to connect to another electronic device, for example, an AR device.

It may be understood that an interface connection relationship between the modules that is shown in this embodiment of the present invention is only an example for description, and does not constitute a limitation on the structure of the electronic device 100. In some other embodiments of this application, the electronic device 100 may alternatively use an interface connection mode different from that in the foregoing embodiment, or use a combination of a plurality of interface connection modes.

The charging management module 140 is configured to receive a charging input from the charger. The charger may be a wireless charger or a wired charger. In some embodiments of wired charging, the charging management module 140 may receive a charging input from a wired charger through the USB port 130. In some embodiments of wireless charging, the charging management module 140 may receive a wireless charging input through a wireless charging coil of the electronic device 100. The charging management module 140 may further supply power to the electronic device through the power management module 141 while charging the battery 142.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes a 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by an audio device (which is not limited to the speaker 170A, the receiver 170B, or the like), or displays an image or a video by the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 100, and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices that integrate at least one communication processing module. The wireless communication module 160 receives an electromagnetic wave by the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 in the electronic device 100 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite-based augmentation system (satellite-based augmentation system, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement a photographing function through the camera 193, the ISP, the video codec, the GPU, the display 194, the application processor and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, and brightness of the image. The ISP may further optimize parameters such as exposure and a color temperature of a photographing scene. In some embodiments, the ISP may be disposed in the camera 193.

The camera 193 is configured to capture a static image or a video. An optical image of an object is generated via the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for converting the electrical signal into a digital image signal. The ISP outputs the digital image signal to the DSP for processing. The DSP converts the digital image signal into an image signal in a standard format, for example, RGB or YUV. In some embodiments, the electronic device 100 may include one or N cameras 193, where N is a positive integer greater than 1.

The digital signal processor is configured to process a digital signal, and may further process another digital signal in addition to a digital image signal. For example, when the electronic device 100 selects a frequency, the digital signal processor is configured to perform Fourier transformation on frequency energy.

The video codec is configured to compress or decompress a digital video. The electronic device 100 may support one or more video codecs. Therefore, the electronic device 100 may play or record videos in a plurality of encoding formats, for example, moving picture experts group (moving picture experts group, MPEG)-1, MPEG-2, MPEG-3, and MPEG-4.

The NPU is a neural-network (neural-network, NN) computing processor, and quickly processes input information with reference to a structure of a biological neural network, for example, a transfer mode between human brain neurons, and may further continuously perform self-learning. Applications such as intelligent cognition of the electronic device 100 may be implemented through the NPU, for example, image recognition, facial recognition, speech recognition, and text understanding.

The external memory interface 120 may be used to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a voice playing function or an image playing function), and the like. The data storage area may store data (such as audio data and a phone book) created during use of the electronic device 100, and the like. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the electronic device 100.

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal output, and is also configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an electrical audio signal into a sound signal. When a call is answered or voice information is received through the electronic device 100, the receiver 170B may be put close to a human ear to listen to a voice.

The microphone 170C, also referred to as a "mike" or a "mic", is configured to convert a sound signal into an electrical signal. When making a call or sending voice information, the user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The headset jack 170D is configured to connect to a wired headset. The headset jack 170D may be a USB port 130, or may be a 3.5 mm open mobile terminal platform (open mobile terminal platform, OMTP) standard interface or cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are many types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch position based on a detection signal of the pressure sensor 180A. In some embodiments, touch operations that are performed in a same touch position but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is performed.

The gyro sensor 180B may be configured to determine a moving posture of the electronic device 100. In some embodiments, an angular velocity of the electronic device 100 around three axes (namely, axes x, y, and z) may be determined through the gyro sensor 180B. The gyro sensor 180B may be configured to implement image stabilization during photographing. For example, when the shutter is pressed, the gyro sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyro sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a flip phone, the electronic device 100 may detect opening and closing of a flip cover by using the magnetic sensor 180D. Further, a feature such as automatic unlocking of the flip cover is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may be further configured to identify a posture of the electronic device, and is used in an application such as switching between a landscape mode and a portrait mode or a pedometer.

The distance sensor 180F is configured to measure a distance. The electronic device 100 may measure the distance in an infrared manner or a laser manner. In some embodiments, in a photographing scene, the electronic device 100 may measure a distance through the distance sensor 180F to implement quick focusing.

The optical proximity sensor 180G may include, for example, a light emitting diode (LED) and an optical detector, for example, a photodiode. The light emitting diode may be an infrared light emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that a user holds the electronic device 100 close to an ear for a call, to automatically perform screen-off for power saving. The optical proximity sensor 180G may also be used in a flip cover mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust a white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy through the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is less than still another threshold, the electronic device 100 boosts an output voltage of the battery 142, to avoid abnormal shutdown due to a low temperature.

The touch sensor 180K is also referred to as a "touch component". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may also be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse to receive a blood pressure beating signal. In some embodiments, the bone conduction sensor 180M may also be disposed in the headset, to obtain a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effect. The motor 191 may also correspond to different vibration feedback effect for touch operations performed on different areas of the display 194. Different application scenarios (for example, a time reminder, information receiving, an alarm clock, and a game) may also correspond to different vibration feedback effect. A touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may be compatible with different types of SIM cards. The SIM card interface 195 is also compatible with an external storage card. The electronic device 100 interacts with a network through the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

A software system of the electronic device 100 may use a layered architecture, an event-driven architecture, a microkernel architecture, a micro service architecture, or a cloud architecture. In this embodiment of the present invention, an Android system of a layered architecture is used as an example to illustrate the software structure of the electronic device 100.

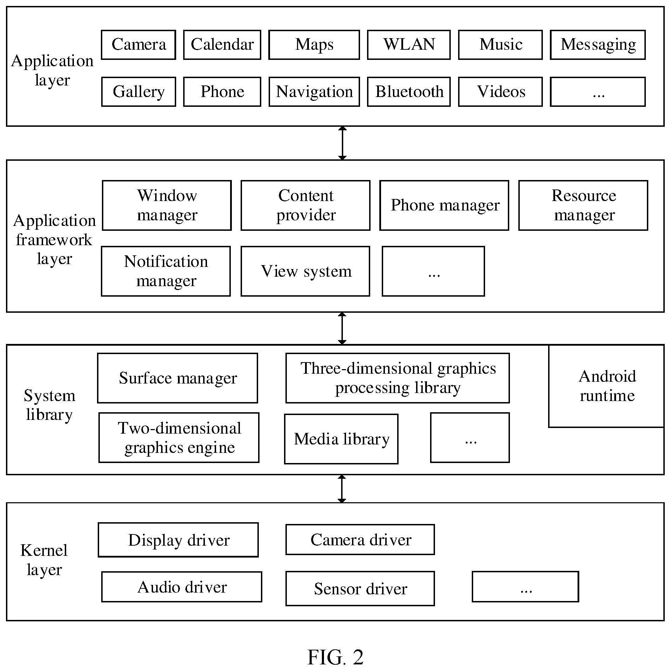

FIG. 2 is a block diagram of a software structure of the electronic device 100 according to an embodiment of the present invention.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 2, an application package may include applications such as Camera, Gallery, Calendar, Calls, Maps, Navigation, WLAN, Bluetooth, Music, Videos, and Messages.

The application framework layer provides an application programming interface (application programming interface, API) and a programming framework for an application at the application layer. The application framework layer includes some predefined functions.

As shown in FIG. 2, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, a phone book, and the like.

The view system includes visual controls such as a control for displaying a text and a control for displaying an image. The view system may be configured to construct an application. A display interface may include one or more views. For example, a display interface including a notification icon of Messages may include a text display view and an image display view.

The phone manager is configured to provide a communication function for the electronic device 100, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification message. The notification manager may automatically disappear after a short pause without requiring a user interaction. For example, the notification manager is configured to notify download completion, give a message notification, and the like. The notification manager may alternatively be a notification that appears in a top status bar of the system in a form of a graph or a scroll bar text, for example, a notification of an application that is run on a background, or may be a notification that appears on the screen in a form of a dialog window. For example, text information is displayed in the status bar, an announcement is given, the electronic device vibrates, or the indicator light blinks.

The Android runtime includes a kernel library and a virtual machine. The Android runtime is responsible for scheduling and management of the Android system.

The kernel library includes two parts: a function that needs to be called in Java language and a kernel library of Android.

The application layer and the application framework layer run on the virtual machine. The virtual machine executes Java files of the application layer and the application framework layer as binary files. The virtual machine is configured to perform functions such as object lifecycle management, stack management, thread management, security and abnormality management, and garbage collection.

The system library may include a plurality of functional modules, for example, a surface manager (surface manager), a media library (Media Library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, static image files, and the like. The media library may support a plurality of audio and video encoding formats, for example, MPEG-4, H.264, MP3, AAC, AMR, JPG, and PNG.

The three-dimensional graphics processing library is configured to implement three-dimensional graphics drawing, image rendering, compositing, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working procedure of software and hardware of the electronic device 100 with reference to a photographing scene.

When the touch sensor 180K receives a touch operation, a corresponding hardware interrupt is sent to the kernel layer. The kernel layer processes the touch operation into an original input event (including information such as touch coordinates and a timestamp of the touch operation). The original input event is stored at the kernel layer. The application framework layer obtains the original input event from the kernel layer, and identifies a control corresponding to the input event. An example in which the touch operation is a touch operation, and a control corresponding to the touch operation is a control of a camera application icon is used. The camera application invokes an interface of the application framework layer to enable the camera application, then enables the camera driver by invoking the kernel layer, and captures a static image or a video through the camera 193.

Ultraviolet (UV) rays in the sun can promote human health, kill bacteria, promote vitamin D generation, and improve nerves, endocrine, immune systems, and the like.

However, excessive ultraviolet exposure and matrix metalloproteinase (MMPs) in the skin can deactivate collagen, damage cells, and cause irreversible effect. Continuous exposure causes skin blackening, sunburn, dry pain, itching, debris, premature aging, and even skin cancer.

The intensity and time of ultraviolet light received by the human body are key parameters for skin health. The ultraviolet intensity and time received by the human body, especially the face, are monitored, and physiological parameters such as an ambient temperature and a body temperature are taken into account, to provide ultraviolet protection/irradiation suggestions and guidance, which is of great significance to human skin health, especially to skin maintenance of female groups.

Currently, a weather APP in the electronic device can provide an ultraviolet index. For example, the weather APP displays an ultraviolet index of 2. Therefore, the weather APP has only a prompt function, and cannot monitor an ultraviolet radiation intensity and time that are actually received by the user. In addition, the user views the weather APP at a low frequency and is unaware of the ultraviolet index.

Currently, a handheld detector has an ultraviolet radiation detection capability. However, the handheld detector can only perform single-point measurement, and cannot be worn, and cannot remind the user in time based on an ultraviolet radiation amount actually received by the user.

Currently, there is another fingernail sticker (with a built-in ultraviolet sensor) for monitoring ultraviolet radiation, and the fingernail sticker is attached to a fingernail of a user to detect ultraviolet rays. Due to frequent movements of both hands of the user, a blocking problem easily exists (for example, the user places the hand in clothes), and ultraviolet radiation actually received by a human body, especially a face, cannot be represented. In addition, there are few physiological signals at the fingernail. The fingernail is far away from the core of the human body, and lacks a key parameter related to radiation, for example, a body temperature.

In conclusion, in the current ultraviolet detection method, a continuously monitoring the ultraviolet radiation intensity received by the human face is not provided, the core body temperature of the human body cannot be accurately detected under the condition of high-intensity ultraviolet radiation, the user cannot be notified to perform ultraviolet protection and temperature protection in time and effectively, and an effective protection reminder cannot be provided for a user.

Based on the foregoing technical problems, an embodiment of the present invention provides an electronic device. The electronic device includes a wearable device that can touch skin of a user. For example, the wearable device includes smart glasses, a smartwatch, or a smart headset. Embodiments of the present invention are applicable to scenarios of ultraviolet radiation monitoring and core body temperature monitoring. The wearable device is used to monitor ultraviolet radiation and a body temperature change of the user, and remind the user in time to perform protection, to protect skin health of the user and avoid a risk such as heatstroke.

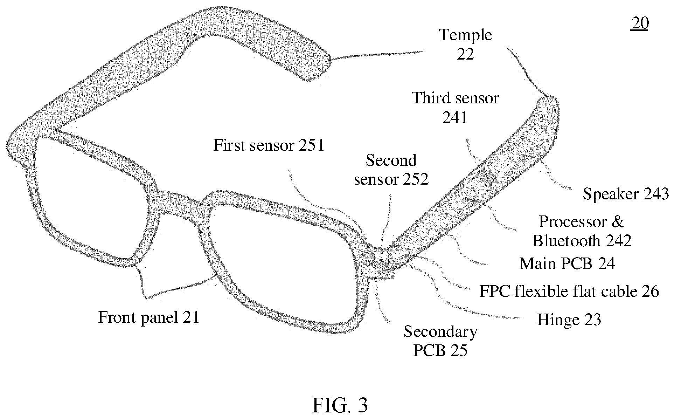

Smart glasses are used as an example. FIG. 3 is a diagram of an electronic device according to an embodiment of the present invention. As shown in FIG. 3, a pair of smart glasses 20 includes a front panel 21 and two temples 22, and the front panel 21 and the temples 22 are movably connected through hinges 23. The front panel 21 includes two lenses. A main PCB 24 is disposed inside one temple 22, and a secondary PCB 25 is disposed inside a position that is on the front panel 21 and that is close to the temple 22. The main PCB 24 and the secondary PCB 25 are electrically connected through an FPC flexible flat cable 26. The FPC flexible flat cable 26 passes through the temple 22 and the hinge 23 (hinge) in a lens frame. A third sensor 241, a processor & Bluetooth 242, and a speaker 243 are disposed on the main PCB 24. A first sensor 251 and a second sensor 252 are disposed on the secondary PCB 25.

The first sensor 251 is an ultraviolet sensor, and is configured to capture an ultraviolet light intensity (also referred to as an ultraviolet index). When the user wears the smart glasses 20, because the first sensor 251 is located in the front panel of the smart glasses 20, the first sensor 251 can capture an ultraviolet light intensity received by the face of the user.

The second sensor 252 is a temperature sensor, is disposed outside the smart glasses 20, and is configured to capture a first temperature. The first temperature is an ambient temperature.

The third sensor 241 is a temperature sensor, is disposed inside the temple 22, can be in contact with the skin of the user, and is configured to capture a second temperature. The second temperature is a skin temperature of the user.

The processor is configured to: obtain the ultraviolet light intensity captured by the first sensor 251, the first temperature captured by the second sensor 252, and the second temperature captured by the third sensor 241; obtain an ultraviolet radiation amount based on the ultraviolet light intensity; obtain a core body temperature of the user based on the ultraviolet light intensity, the first temperature, and the second temperature; and provide a protection prompt for the user based on the ultraviolet radiation amount and the core body temperature.

Bluetooth is used to establish a Bluetooth connection to another electronic device.

The speaker 243 is configured to play a protection prompt voice.

In an optional solution, the processor is specifically configured to perform integral calculation on the ultraviolet light intensity to obtain the ultraviolet radiation amount. The user continuously receives ultraviolet radiation in an ultraviolet radiation scenario, and the ultraviolet radiation amount U continuously received by the user is calculated according to a method of performing integration on an ultraviolet light intensity Uv detected by the ultraviolet sensor:

$$U = \int U_v dt.$$

In another optional solution, the processor is specifically configured to perform cumulative summation on the ultraviolet light intensity to obtain the ultraviolet radiation amount. During actual monitoring, the ultraviolet sensor is invoked once at an interval of $t_i$, the detected ultraviolet light intensity is $U_{Vi}$, and the ultraviolet radiation amount U is calculated in a cumulative summation manner:

$$U = \sum U_{vi} t_i.$$

The processor is specifically configured to: obtain the ultraviolet light intensity captured by the first sensor; and adjust an invocation frequency of the first sensor based on the ultraviolet light intensity. The user pays more attention to an amount of received strong ultraviolet radiation (for example, $U_{Vi} > 5$). Therefore, the invocation frequency of the first sensor may be dynamically adjusted based on the ultraviolet light intensity detected by the first sensor, so that power consumption of the device can be effectively reduced, a battery life of the wearable device can be increased, and continuous low-power monitoring for a long time can be implemented. As shown in Table 1, lower $U_{Vi}$ indicates a lower invocation frequency.

Table 1 Invocation frequencies that are of the first sensor and that correspond to different ultraviolet light intensities

| $U_{Vi}$ | Invocation frequency of the first sensor |
|---|---|
| 0 to 2 | Once every 10 minutes |
| 3 to 5 | Once every 5 minutes |
| 6 to 7 | Once every minute |
| 8 to 10 | Once every 30s |
| 11+ | Once every 10s |

The processor is specifically configured to: if the ultraviolet light intensity is less than a first threshold, obtain the core body temperature of the user based on the first temperature and the second temperature. Specifically, the processor calculates the first temperature and the second temperature by using a multiple linear regression model, to obtain the core body temperature.

In this embodiment of the present invention, a current ultraviolet radiation degree is determined based on the ultraviolet light intensity. When the ultraviolet light intensity is less than the first threshold, it indicates that the user is in a low ultraviolet radiation scenario, and the ultraviolet light has small impact on the core body temperature of the user. Therefore, when the core body temperature is calculated, the ultraviolet light intensity may not be considered, and only an ambient temperature and a skin temperature of the user are considered.

The first threshold is a preset ultraviolet light intensity threshold.

The multiple linear regression model is $T_C = f(T_E, T_S, c1)$, c1 is a constant, $T_E$ is the first temperature, $T_S$ is the second temperature, and $T_C$ is the core body temperature.

The processor is specifically configured to: if the ultraviolet light intensity is greater than or equal to the first threshold, obtain the core body temperature of the user based on the ultraviolet light intensity, the first temperature, and the second temperature. Specifically, the processor calculates the ultraviolet light intensity, the first temperature, and the second temperature by using an XGBoost model, to obtain the core body temperature.

When the ultraviolet light intensity is greater than or equal to the first threshold, it indicates that the user is in a scenario with high ultraviolet radiation, and the ultraviolet light has large impact on the core body temperature of the user. Therefore, the ultraviolet light intensity needs to be considered when the core body temperature is calculated.

The XGBoost model is $T_C = f(T_E, T_S, U_{VI}, c2)$, c2 is a constant, $T_E$ is the first temperature, $T_S$ is the second temperature, $U_{VI}$ is the ultraviolet light intensity, and $T_C$ is the core body temperature.

When the human body is exposed to strong ultraviolet radiation, both the skin temperature of the human head and the ambient temperature of the environment in which the smart glasses 20 are located increase greatly. However, due to a body temperature adjustment mechanism of the human body, the core body temperature increases slightly. If only the skin temperature and the ambient temperature are used to calculate the core body temperature without considering the ultraviolet radiation intensity, a large deviation may occur, resulting in an incorrect body temperature exception reminder. In the case of strong ultraviolet radiation, the coupling introduced by the ultraviolet light intensity can be added based on the skin temperature and the ambient temperature to accurately calculate the core temperature and provide an accurate result for the user. In this way, the ultraviolet light intensity is compared with the first threshold, and core body temperature detection models are separately established based on scenarios with radiation intensities, so that accuracy of core body temperature detection can be effectively improved. A method for calculating the core body temperature in scenarios with different ultraviolet radiation is implemented.

The processor is specifically configured to provide an ultraviolet protection prompt for the user based on the ultraviolet radiation amount. Specifically, if the ultraviolet radiation amount is less than or equal to a second threshold, it indicates that the user receives a small ultraviolet radiation amount, and the processor skips providing an ultraviolet protection prompt. If the ultraviolet radiation amount is greater than the second threshold, it indicates that the user receives a large ultraviolet radiation amount, and the processor provides an ultraviolet protection prompt for the user based on the ultraviolet radiation amount.

In this embodiment of the present invention, the second threshold is an ultraviolet radiation amount threshold.

Optionally, the second threshold is set by the user. Before the processor obtains the ultraviolet light intensity captured by the first sensor 251, the first temperature captured by the second sensor 252, and the second temperature captured by the third sensor 241, the processor is further configured to obtain the second threshold in response to a change operation performed by the user on a target control used to display the second threshold.

Optionally, the second threshold is a personalized threshold corresponding to a physiological parameter of the user. For example, the mobile phone generates the personalized threshold based on the physiological parameter of the user, and then the smart glasses obtain the personalized threshold from the mobile phone as the second threshold. Optionally, the mobile phone detects skin roughness and an aging degree by photographing skin of a face or an arm of the user, and generates the personalized threshold. Optionally, the user may manually change the personalized threshold based on a requirement of daily life and a working scenario of the user.

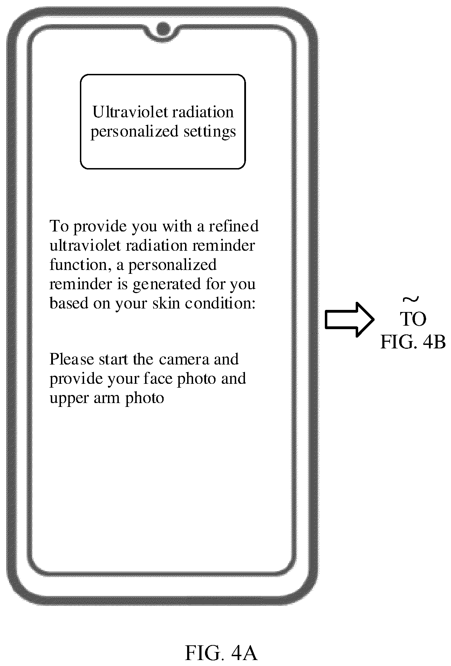

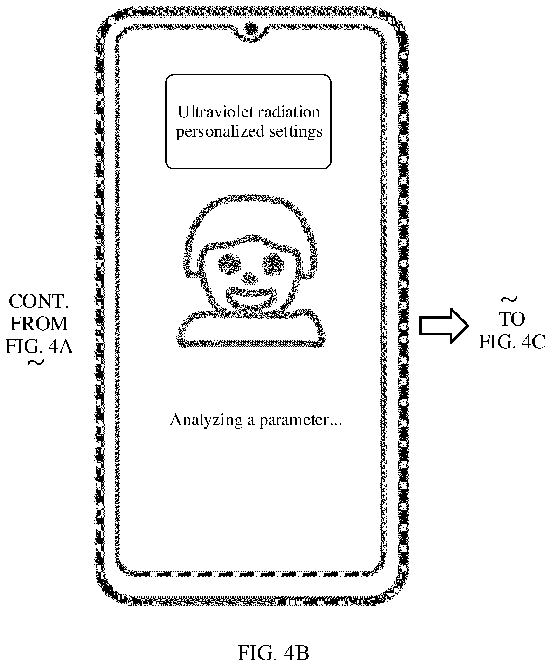

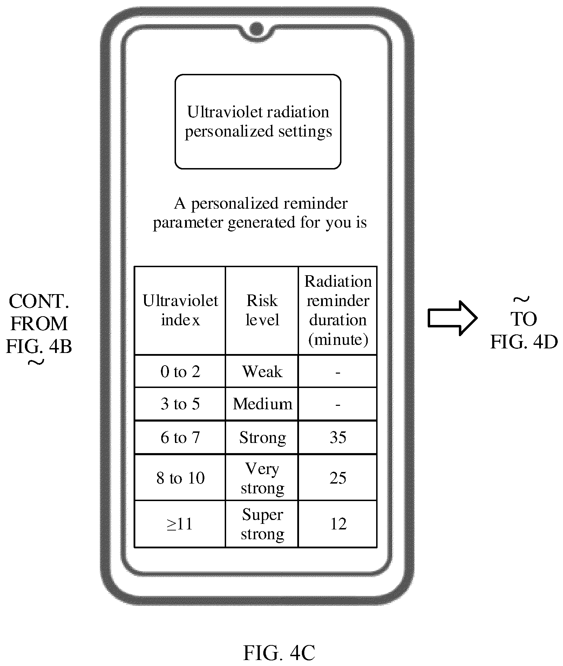

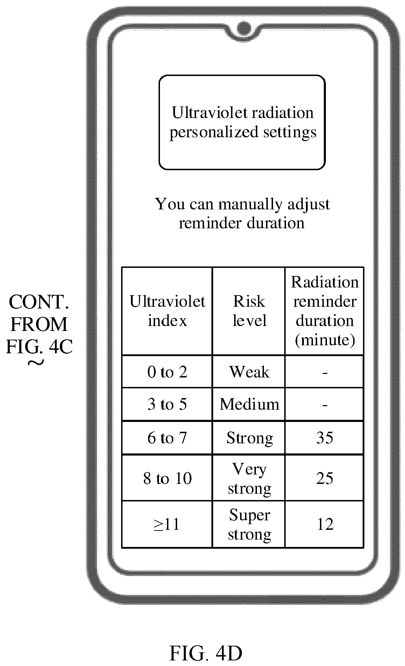

When the ultraviolet radiation amount received by the human body exceeds the second threshold, the user needs to be reminded in time to pay attention to protection. Due to different physiques of people and different capabilities of bearing ultraviolet radiation, the second threshold may be adaptively set based on a skin status, a roughness degree, and the like of the user, to set a personalized parameter. FIG. 4A to FIG. 4D are a diagram of generating a personalized threshold by a mobile phone based on a physiological parameter of a user according to this embodiment of the present invention. As shown in FIG. 4A to FIG. 4D, the mobile phone displays an ultraviolet radiation personalized settings interface, and first prompts the user to start a camera and provide a face photo and an upper arm photo. After the user provides the face photo and the upper arm photo, an interface for parameter analysis is displayed. In this case, the mobile phone performs parameter analysis on the face photo and the upper arm photo, and recognizes physiological parameters such as skin roughness and an aging degree of the user by using the face photo and the upper arm photo, comprehensive analysis is performed based on the physiological parameter of the user. After the parameter analysis of the mobile phone is completed, the mobile phone displays the generated personalized reminder parameter. The personalized reminder parameter includes radiation reminder duration corresponding to different ultraviolet ray intensities. The personalized threshold is ultraviolet radiation amounts in different ultraviolet radiation indices and corresponding radiation reminder duration. In addition, the mobile phone may further prompt the user to manually adjust the radiation reminder duration, to achieve a purpose of changing the personalized threshold. This embodiment of the present invention provides a capability of setting the second threshold in a personalized manner, to meet a requirement of a wider use group. For example, a user who works outdoors with high ultraviolet radiation for a long time may adjust the second threshold based on a requirement of the user.

The processor is specifically configured to provide a temperature protection prompt for the user based on the core body temperature. Specifically, if the core body temperature is greater than or equal to a third threshold and less than or equal to a fourth threshold, the processor skips providing a temperature protection prompt; if the core body temperature is less than the third threshold, the processor provides a prompt of paying attention to hypothermia protection for the user based on the core body temperature; or if the core temperature is greater than the fourth threshold, the processor provides a prompt of paying attention to heatstroke prevention for the user based on the core body temperature.

In this embodiment of the present invention, a manner of providing the ultraviolet protection prompt and/or the temperature protection prompt for the user includes one or any combination of the following options: automatically changing a color of a lens, displaying prompt content on the lens with a display function, playing the prompt content through a speaker 243, and providing the ultraviolet protection prompt and/or the temperature protection prompt through another electronic device.

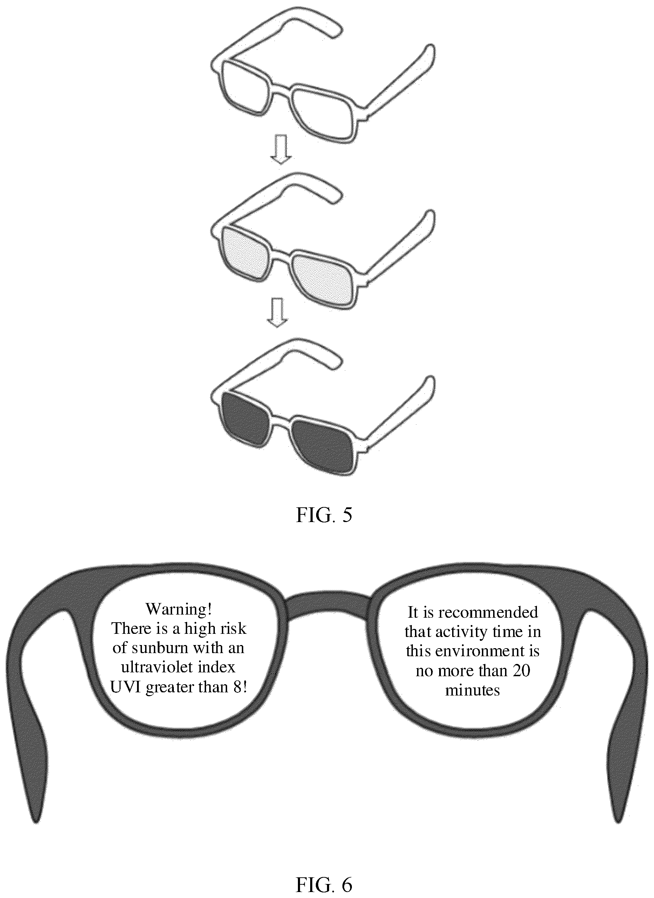

Optionally, a lens of the smart glasses 20 is an electrochromic lens. The processor is further configured to adjust an applied voltage of an electrochromic material in the lens based on the ultraviolet light intensity. The smart glasses 20 monitor the ultraviolet light intensity in real time, and when the ultraviolet light intensity exceeds a specified threshold, the user is reminded by using an electrochromic lens. Specifically, the applied voltage V of the electrochromic material is adjusted based on the ultraviolet light intensity, where $V=V_0+k*U_v$, $V_0$ and k are constants, and $U_v$ is the ultraviolet light intensity. FIG. 5 is a diagram in which the color of the lens becomes deeper as the applied voltage increases according to this embodiment of the present invention. As shown in FIG. 5, a larger Uv indicates a stronger applied voltage V, a deeper color of the lens, and a stronger prompt degree for the user. The smart glasses 20 automatically darken the color of the lens when the user receives a higher ultraviolet light intensity. In embodiments of the present invention, the color of the lens is automatically adjusted, so that the user can be directly and intuitively prompted, and an ultraviolet radiation amount received by the eyes of the user can be reduced, thereby protecting the eyes.

Optionally, the lens of the smart glasses 20 has a display function. The processor is further configured to display prompt content on the lens based on the ultraviolet light intensity. The smart glasses 20 may interact with the user and provide a protection prompt by displaying a text on the lens. For example, the smart glasses 20 monitor the ultraviolet light intensity in real time, and when the ultraviolet light intensity exceeds the specified threshold, a current ultraviolet index and text reminder content are displayed on the lens. For example, FIG. 6 is a diagram of prompt content displayed on a lens according to this embodiment of the present invention. As shown in FIG. 6, "Warning! There is a high risk of sunburn with an ultraviolet index UVI greater than 8! The message "It is recommended that activity time in this environment is no more than 20 minutes" is displayed on the right lens. In this embodiment of the present invention, the prompt content may be presented to the user in a quantitative form, and the user may perform targeted protection.

The smart glasses 20 include a speaker 243. Optionally, the smart glasses 20 play the prompt content through the speaker 243. The smart glasses 20 remind the user and provides a protection suggestion in a form of voice broadcast through a speaker based on the ultraviolet index and the core body temperature that are monitored in real time, so that a field of view of the user can be not affected through voice interaction.

The smart glasses 20 transfer the prompt content to a terminal, for example, a mobile phone or a smartwatch in a wireless transmission manner, for example, Bluetooth, and display the reminder and a protection suggestion in a form of vibration or a text/picture.

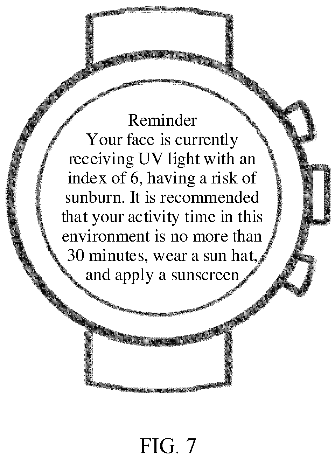

For example, FIG. 7 is a diagram of displaying prompt content by a smartwatch according to an embodiment of the present invention. As shown in FIG. 7, the smartwatch displays the prompt content in a text form, the prompt content includes a title and text information, and the title is "Reminder". The text information is "Your face is currently receiving ultraviolet light with an index of 6, having a risk of sunburn. It is recommended that you stay in this environment for no more than 30 minutes, wear a sun hat, and apply a sunscreen".

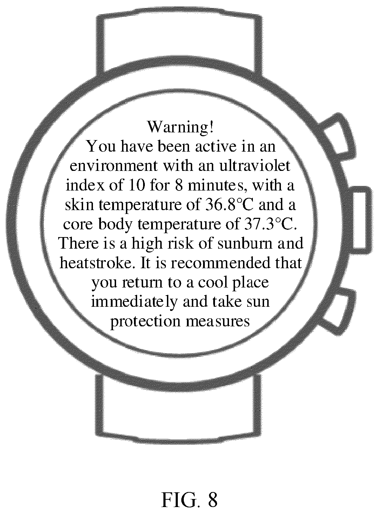

For example, FIG. 8 is another diagram of displaying prompt content by a smartwatch according to this embodiment of the present invention. As shown in FIG. 8, the smartwatch displays the prompt content in a text form, the prompt content includes a title and text information, and the title is "Reminder". The text information is "You have been active in an environment with an ultraviolet index of 10 for 8 minutes, with a skin temperature of 36.8°C, and a core body temperature of 37.3°C. There is a high risk of sunburn and heatstroke. It is recommended that you return to a cool place immediately and take sun protection measures".

In this embodiment of the present invention, the watch vibrates, so that a strong reminder can be implemented. In addition, a watch face of the watch can be turned up and down to display more text or picture content.

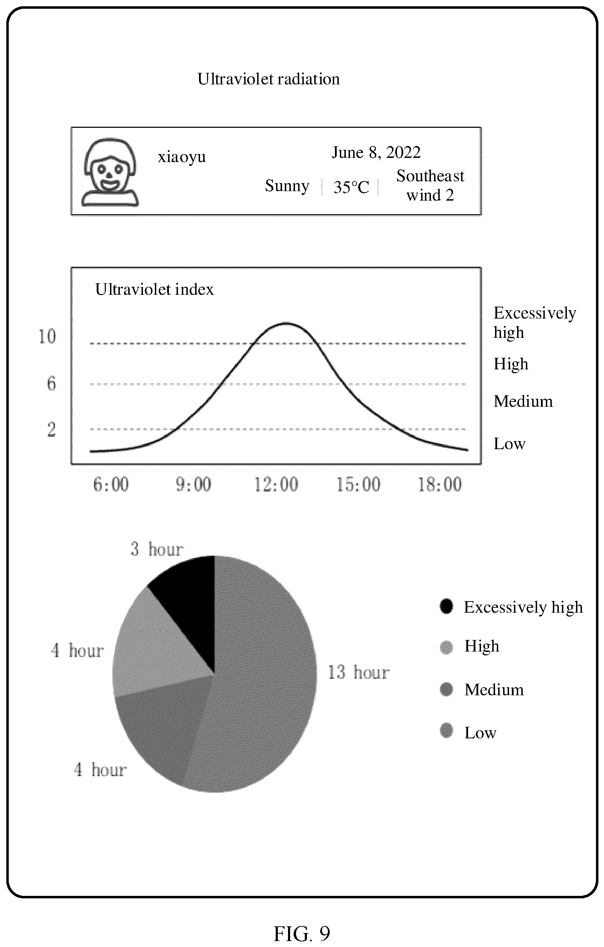

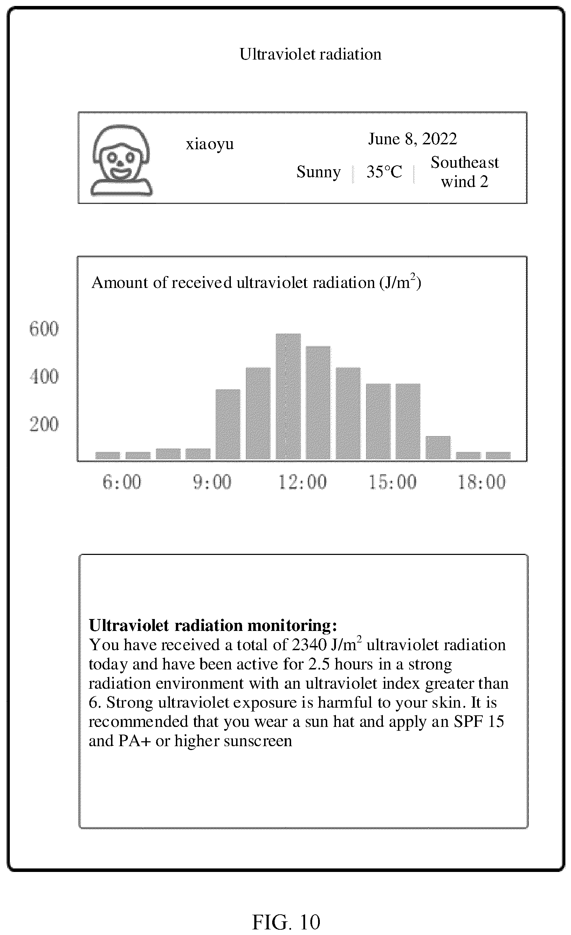

For example, FIG. 9 is a diagram of displaying an ultraviolet radiation monitoring interface by a mobile phone according to this embodiment of the present invention; and FIG. 10 is a diagram of an ultraviolet radiation monitoring interface after a user performs a slide-up operation in FIG. 9. On the ultraviolet radiation monitoring interfaces shown in FIG. 9 and FIG. 10, the smart glasses record ultraviolet radiation information in a monitoring APP on the mobile phone. The ultraviolet radiation information includes an ultraviolet index and an amount of received ultraviolet radiation that are received in different time periods, time proportion of each ultraviolet radiation amount in a day, a summary record, and a suggestion. As shown in FIG. 9, the ultraviolet index received in different time periods may be displayed in a form of a curve chart, and a time proportion of each ultraviolet radiation amount in a day may be displayed in a form of a pie chart. As shown in FIG. 10, the amount of received ultraviolet radiation may be displayed in a form of a column chart. The summary record and the suggestion are as follows: "Ultraviolet radiation monitoring: You have received a total of 2340 J/m$^2$ ultraviolet radiation today and have been active for 2.5 hours in an environment with an ultraviolet index greater than 6. Strong ultraviolet exposure is harmful to your skin. It is recommended that you wear a sun hat and apply an SPF 15 and PA+ or higher sunscreen." In this embodiment of the present invention, a detailed process in which a parameter, for example, an ultraviolet radiation amount changes with time may be performed on a mobile phone page, and a user may search for a historical record to obtain an accumulated radiation amount in a recent period of time. In addition, the mobile phone records more detailed content, so that a user habit can be analyzed based on a historical data change, and a comprehensive and better suggestion and protection measure can be provided.

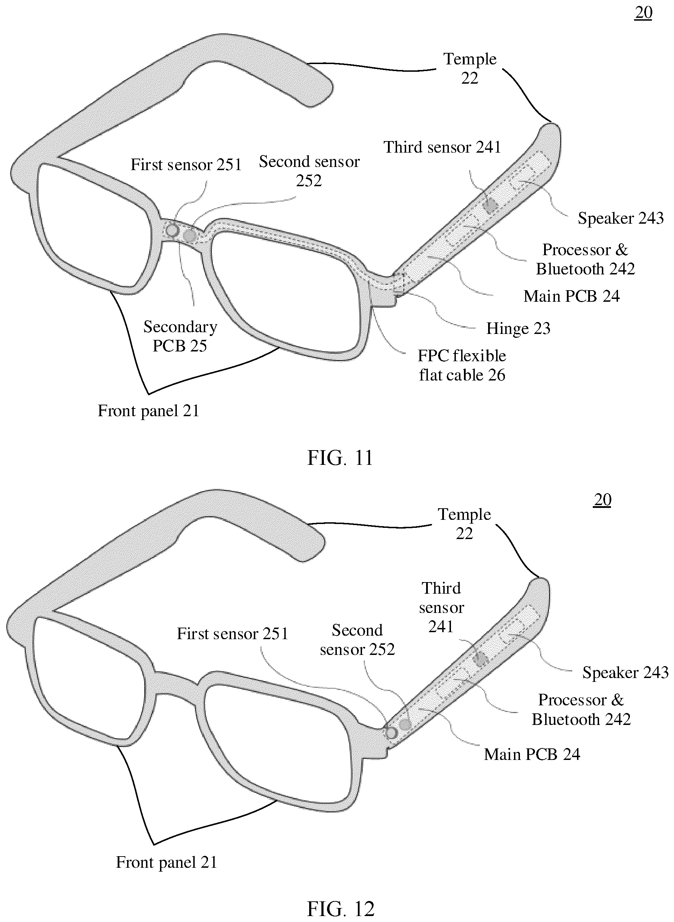

In the smart glasses 20 shown in FIG. 3, the secondary PCB 25, the first sensor 251, and the second sensor 252 are located in positions that are in the front panel 21 of the lens frame and that are close to the temple 22. Further, an embodiment of the present invention provides still another electronic device. FIG. 11 is a diagram of another electronic device according to an embodiment of the present invention. As shown in FIG. 11, compared with the smart glasses 20 shown in FIG. 3, the secondary PCB 25, the first sensor 251, and the second sensor 252 in the smart glasses 20 shown in FIG. 11 are located in middle positions of the front panel 21 of the lens frame. When a user wears the smart glasses 20 shown in FIG. 11, because the first sensor 251 is located right in front of the face, and an ultraviolet light intensity received by the first sensor is completely consistent with an ultraviolet light intensity received by the face, an ultraviolet radiation amount received by the face can be more accurately evaluated, and a monitoring result is more accurate.

Further, an embodiment of the present invention provides still another electronic device. FIG. 12 is a diagram of another electronic device according to an embodiment of the present invention. As shown in FIG. 12, compared with the smart glasses 20 shown in FIG. 3 and FIG. 11, the smart glasses 20 shown in FIG. 12 do not include the secondary PCB 25 and the FPC flexible flat cable 26, in addition, the first sensor 251 and the second sensor 252 are both located on the main PCB 24 inside the temple 22. The first sensor 251 and the second sensor 252 are located in positions that are in the temple 22 and that are close to the front panel 21. A hardware layout of the smart glasses 20 shown in FIG. 12 is simpler, and an FPC flexible flat cable passing through a hinge does not need to be used, so that hardware overheads can be reduced. In addition, although the ultraviolet light intensity received by the first sensor 251 is slightly different from an ultraviolet light intensity on the front face, the ultraviolet light intensity may also be corrected according to an algorithm.

Based on the electronic device shown in FIG. 3, FIG. 11, and FIG. 12, an embodiment of the present invention provides a protection prompt method.

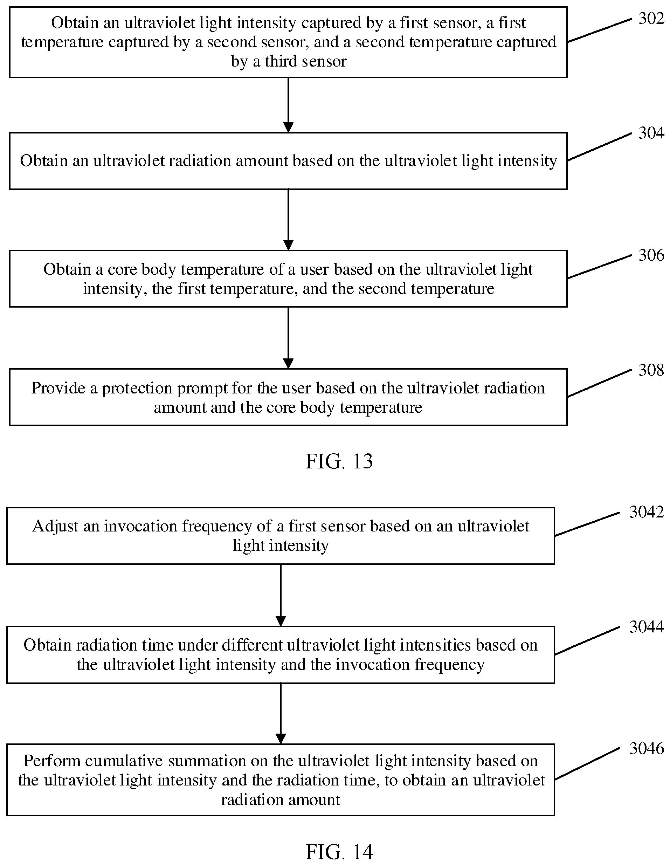

FIG. 13 is a flowchart of a protection prompt method according to an embodiment of the present invention. As shown in FIG. 13, the method includes the following steps.

Step 302: Obtain an ultraviolet light intensity captured by a first sensor, a first temperature captured by a second sensor, and a second temperature captured by a third sensor.

In this embodiment of the present invention, the first temperature is an ambient temperature, and the second temperature is a skin temperature of a user.

Optionally, the first sensor and the second sensor are located in positions that are in a temple of the smart glasses and that are close to a front panel.

Optionally, the first sensor and the second sensor are located on a front panel of a lens frame of the smart glasses. The first sensor and the second sensor are located in positions that are close to a temple and that are in the front panel of the lens frame, or the first sensor and the second sensor are located in middle positions on the front panel of the lens frame.

The second sensor is located outside the smart glasses, and the third sensor is located inside the temple of the smart glasses.

Step 304: Obtain an ultraviolet radiation amount based on the ultraviolet light intensity.

In this embodiment of the present invention, as shown in FIG. 14, step 304 specifically includes the following steps.

Step 3042: Adjust an invocation frequency of the first sensor based on the ultraviolet light intensity.

Step 3044: Obtain radiation time at different ultraviolet light intensities based on the ultraviolet light intensity and the invocation frequency.

Step 3046: Perform cumulative summation on the ultraviolet light intensity based on the ultraviolet light intensity and the radiation time, to obtain the ultraviolet radiation amount.

Step 306: Obtain a core body temperature of the user based on the ultraviolet light intensity, the first temperature, and the second temperature.

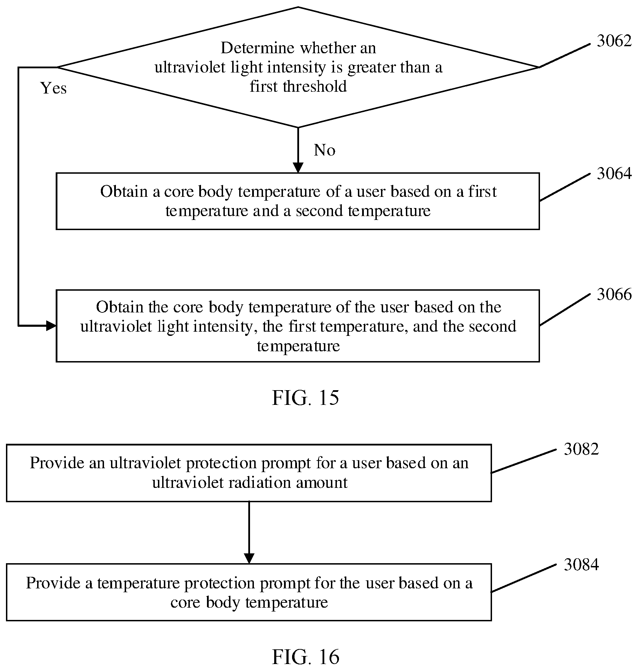

In this embodiment of the present invention, as shown in FIG. 15, step 306 specifically includes the following steps.

Step 3062: Determine whether the ultraviolet light intensity is greater than a first threshold; and if the ultraviolet light intensity is greater than the first threshold, perform step 3066; or if the ultraviolet light intensity is not greater than the first threshold, perform step 3064.

Step 3064: Obtain the core body temperature of the user based on the first temperature and the second temperature, and continue to perform step 308.

If the ultraviolet light intensity is less than the first threshold, the core body temperature of the user is obtained based on the first temperature and the second temperature. Step 3064 specifically includes: calculating the first temperature and the second temperature by using a multiple linear regression model, to obtain the core body temperature.

Step 3066: Obtain the core body temperature of the user based on the ultraviolet light intensity, the first temperature, and the second temperature, and continue to perform step 308.

If the ultraviolet light intensity is greater than or equal to a first threshold, the core body temperature of the user is obtained based on the ultraviolet light intensity, the first temperature, and the second temperature. Step 3066 specifically includes: calculating the ultraviolet light intensity, the first temperature, and the second temperature by using an XGBoost model, to obtain the core body temperature.

Step 308: Provide a protection prompt for the user based on the ultraviolet radiation amount and the core body temperature.

In this embodiment of the present invention, as shown in FIG. 16, step 308 includes the following step.

Step 3082: Provide the ultraviolet protection prompt for the user based on the ultraviolet radiation amount.

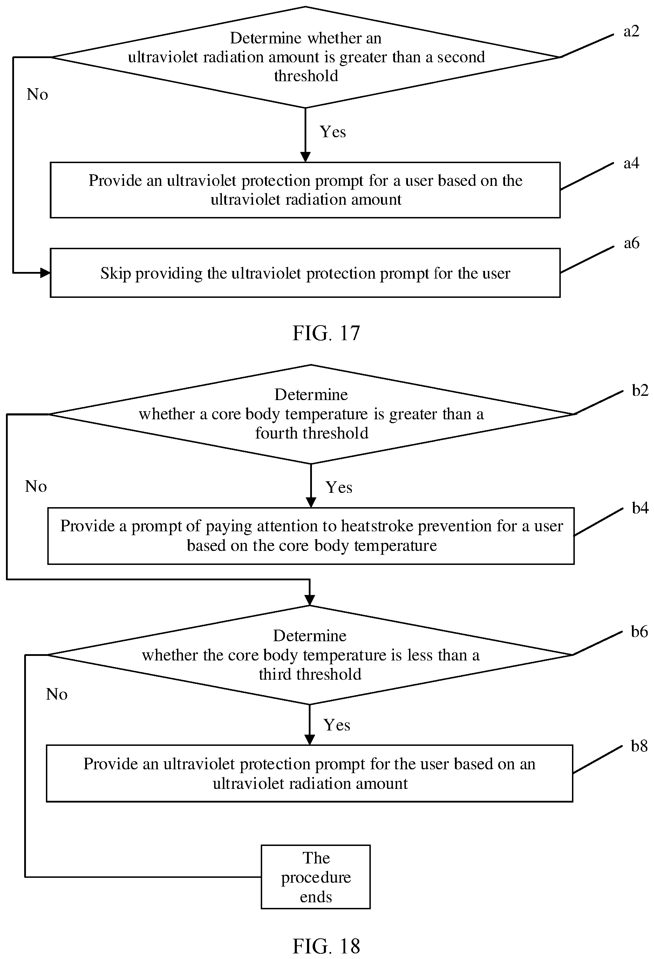

As shown in FIG. 17, step 3082 specifically includes the following steps.

Step a2: Determine whether the ultraviolet radiation amount is greater than a second threshold; and if the ultraviolet radiation amount is greater than the second threshold, perform step a4; or if the ultraviolet radiation amount is not greater than the second threshold, perform step a6.

Step a4: Provide an ultraviolet protection prompt for the user based on the ultraviolet radiation amount, and continue to perform step 3084.

Step a6: Skip providing the ultraviolet protection prompt for the user, and continue to perform step 3084.

In this embodiment of the present invention, if the ultraviolet radiation amount is less than or equal to the second threshold, it indicates that the user receives a small ultraviolet radiation amount, and no ultraviolet protection prompt is provided, and step 3084 continues to be performed. If the ultraviolet radiation amount is greater than the second threshold, it indicates that the user receives a large ultraviolet radiation amount, and the ultraviolet protection prompt is provided for the user based on the ultraviolet radiation amount.

Optionally, the second threshold is a personalized threshold corresponding to a physiological parameter of the user.

Optionally, before step 302, the method further includes: obtaining the second threshold in response to a change operation performed by the user on a target control used to display the second threshold.

Step 3084: Provide a temperature protection prompt for the user based on the core body temperature.

As shown in FIG. 18, step 3084 specifically includes the following steps.

Step b2: Determine whether the core body temperature is greater than a fourth threshold; and if the core body temperature is greater than the fourth threshold, perform step b4; or if the core body temperature is not greater than the fourth threshold, perform step b6.

Step b4: Provide a prompt of paying attention to heatstroke prevention for the user based on the core body temperature, and the procedure ends.

If the core temperature is greater than the fourth threshold, the prompt of paying attention to heatstroke prevention is provided for the user based on the core body temperature.

Step b6: Determine whether the core body temperature is less than a third threshold; and if the core body temperature is less than the third threshold, perform step b8; or if the core body temperature is not less than the third threshold, the procedure ends.

Step b8: Provide a prompt of paying attention to hypothermia protection for the user based on the core body temperature, and the procedure ends.

If the core body temperature is less than the third threshold, the prompt of paying attention to hypothermia protection is provided for the user based on the core body temperature.

If the core body temperature is greater than or equal to the third threshold and less than or equal to the fourth threshold, no temperature protection prompt is provided.

A manner of providing the ultraviolet protection prompt and/or the temperature protection prompt for the user includes one or any combination of the following options: automatically changing a color of a lens, displaying prompt content on the lens with a display function, playing the prompt content through a speaker, and providing the ultraviolet protection prompt and/or the temperature protection prompt through another electronic device.

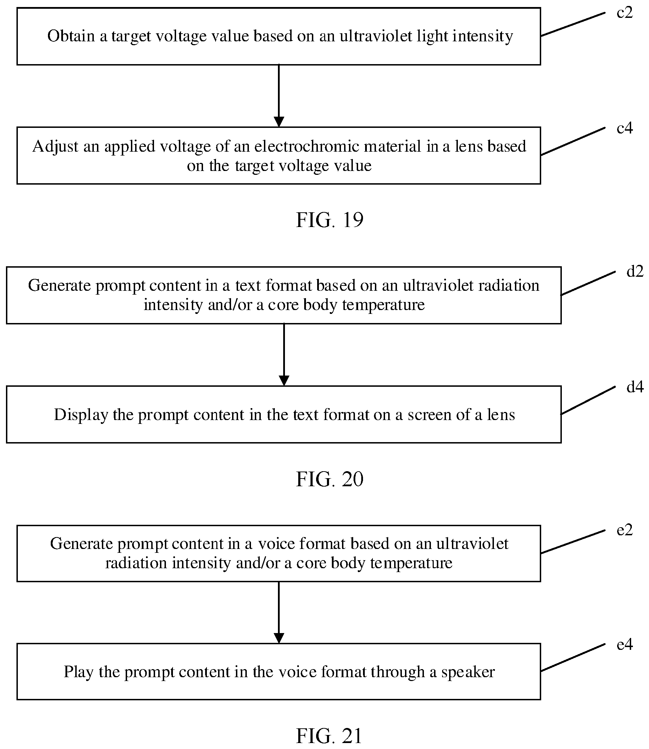

As shown in FIG. 19, automatically changing a color of a lens includes the following steps.

Step c2: Obtain a target voltage value based on the ultraviolet light intensity.

The target voltage value $V = V_0 + k*U_v$, where $V_0$ and $k$ are constants, and $U_v$ is the ultraviolet light intensity.

Step c4: Adjust an applied voltage of an electrochromic material in the lens based on the target voltage value.

In this step, the applied voltage of the electrochromic material is the target voltage value V.

In this embodiment of the present invention, the applied voltage of the electrochromic material in the lens is adjusted based on the ultraviolet light intensity, to change the color of the lens. When the ultraviolet light intensity received by the user is higher, the color of the lens is darkened, so that the user can be directly and intuitively prompted, and an ultraviolet radiation amount received by the eyes of the user can be reduced. This can protect the eyes.

As shown in FIG. 20, displaying prompt content on the lens with a display function includes the following steps.

Step d2: Generate the prompt content in a text format based on the ultraviolet radiation intensity and/or the core body temperature.

Step d4: Display the prompt content in the text format on a screen of the lens.

As shown in FIG. 21, playing the prompt content through a speaker includes the following steps.

Step e2: Generate the prompt content in a voice format based on the ultraviolet radiation intensity and/or the core body temperature.

Step e4: Play the prompt content in the voice format through the speaker.

In the technical solution of the body temperature and ultraviolet radiation monitoring method provided in this embodiment of the present invention, the electronic device includes a first sensor, a second sensor, and a third sensor. The electronic device obtains an ultraviolet light intensity captured by the first sensor, a first temperature captured by the second sensor, and a second temperature captured by the third sensor; obtains an ultraviolet radiation amount based on the ultraviolet light intensity; obtains a core body temperature of a user based on the ultraviolet light intensity, the first temperature, and the second temperature; and provides a protection prompt for the user based on the ultraviolet radiation amount and the core body temperature. This provides a continuous monitoring capability and accurately detects the core body temperature of the human body, thereby providing an effective protection reminder for the user.

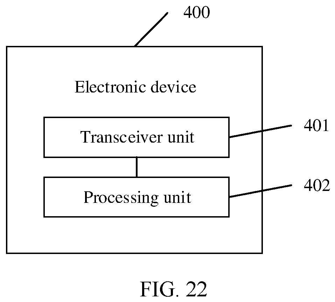

FIG. 22 is a diagram of a structure of an electronic device according to an embodiment of the present invention. It should be understood that the electronic device 400 can perform steps of the electronic device in the foregoing protection prompt method. To avoid repetition, details are not described herein. The electronic device 400 includes a transceiver unit 401 and a processing unit 402.

The transceiver unit 401 is configured to obtain an ultraviolet light intensity captured by the first sensor, a first temperature captured by the second sensor, and a second temperature captured by the third sensor.

The processing unit 402 is configured to: obtain an ultraviolet radiation amount based on the ultraviolet light intensity; obtain a core body temperature of a user based on the ultraviolet light intensity, the first temperature, and the second temperature; and provide a protection prompt for the user based on the ultraviolet radiation amount and the core body temperature.

Optionally, the processing unit 402 is specifically configured to perform cumulative summation on the ultraviolet light intensity to obtain the ultraviolet radiation amount.

Optionally, the processing unit 402 is specifically configured to: if the ultraviolet light intensity is less than a first threshold, obtain the core body temperature of the user based on the first temperature and the second temperature; or if the ultraviolet light intensity is greater than or equal to the first threshold, obtain the core body temperature of the user based on the ultraviolet light intensity, the first temperature, and the second temperature.

Optionally, the processing unit 402 is specifically configured to calculate the first temperature and the second temperature by using a multiple linear regression model, to obtain the core body temperature.

Optionally, the processing unit 402 is specifically configured to calculate the ultraviolet light intensity, the first temperature, and the second temperature by using an XGBoost model, to obtain the core body temperature.

Optionally, the processing unit 402 is specifically configured to: provide an ultraviolet protection prompt for the user based on the ultraviolet radiation amount; and provide a temperature protection prompt for the user based on the core body temperature.

Optionally, the processing unit 402 is specifically configured to: if the ultraviolet radiation amount is less than or equal to a second threshold, skip providing the ultraviolet protection prompt; or if the ultraviolet radiation amount is greater than the second threshold, provide the ultraviolet protection prompt for the user based on the ultraviolet radiation amount.

Optionally, the processing unit 402 is specifically configured to: if the core body temperature is greater than or equal to a third threshold and less than or equal to a fourth threshold, skip providing the temperature protection prompt; if the core body temperature is less than the third threshold, provide a prompt of paying attention to hypothermia protection for the user based on the core body temperature; or if the core temperature is greater than the fourth threshold, provide a prompt of paying attention to heatstroke prevention for the user based on the core body temperature.

Optionally, the second threshold is a personalized threshold corresponding to a physiological parameter of the user.

Optionally, before the transceiver unit 401 obtains the ultraviolet light intensity captured by the first sensor, the first temperature captured by the second sensor, and the second temperature captured by the third sensor, the processing unit 402 is further configured to: obtain the second threshold in response to a change operation performed by the user on a target control used to display the second threshold.

Optionally, the first temperature is an ambient temperature, and the second temperature is a skin temperature of the user.

Optionally, the electronic device includes smart glasses.

Optionally, a manner of providing the ultraviolet protection prompt and/or the temperature protection prompt for the user includes one or any combination of the following options: automatically changing a color of a lens, displaying prompt content on the lens with a display function, playing the prompt content through a speaker, and providing the ultraviolet protection prompt and/or the temperature protection prompt through another electronic device.

Optionally, the smart glasses include an electrochromic lens.

Optionally, the processing unit 402 is specifically configured to adjust an applied voltage of an electrochromic material in the lens based on the ultraviolet light intensity.

Optionally, the transceiver unit 401 is specifically configured to obtain the ultraviolet light intensity captured by the first sensor; and; and the processing unit 402 is specifically configured to adjust an invocation frequency of the first sensor based on the ultraviolet light intensity.

Optionally, the first sensor and the second sensor are located in positions that are in a temple of the smart glasses and that are close to a front panel.

Optionally, the first sensor and the second sensor are located on a front panel of a lens frame of the smart glasses.

Optionally, the first sensor and the second sensor are located in positions that are close to a temple and that are in the front panel of the lens frame.

Optionally, the first sensor and the second sensor are located in middle positions of the front panel of the lens frame.

Optionally, the second sensor is located outside the smart glasses, and the third sensor is located inside the temple of the smart glasses.

Optionally, the electronic device includes a smart watch or a smart headset.

It should be understood that the electronic device 400 herein is implemented in a form of a functional unit. The term "unit" herein may be implemented in a form of software and/or hardware. This is not specifically limited. For example, the "unit" may be a software program, a hardware circuit, or a combination thereof that implements the foregoing function. The hardware circuit may include an application-specific integrated circuit (application-specific integrated circuit, ASIC), an electronic circuit, a processor (for example, a shared processor, a dedicated processor, or a group processor) configured to execute one or more software or firmware programs, a memory, a merged logic circuit, and/or another appropriate assembly that supports the described function.

Therefore, the units in the examples described in embodiments of the present invention can be implemented by using electronic hardware, or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of the present invention.

An embodiment of this application provides an electronic device. The electronic device may be a terminal device or a circuit device built into the terminal device. The electronic device may be configured to perform the functions/steps in the foregoing method embodiments.

An embodiment of this application provides a computer-readable storage medium. The computer-readable storage medium stores instructions. When the instructions are run on a terminal device, the terminal device is enabled to perform the functions/steps in the foregoing method embodiments.

An embodiment of this application further provides a computer program product including instructions. When the computer program product runs on a computer or any at least one processor, the computer is enabled to perform the functions/steps in the foregoing method embodiments.

In embodiments of this application, "at least one" means one or more, and "a plurality of" means two or more. For example, A and/or B may represent the following three cases: Only A exists, both A and B exist, and only B exists. A and B may be singular or plural. The character "/" usually indicates an "or" relationship between the associated objects. "At least one of the following" and similar expressions refer to any combination of these terms, including any combination of single or plural terms. For example, at least one of a, b, and c may represent: a, b, c, a-b, a-c, b-c, or a-b-c, where a, b, and c may be one or more.

A person of ordinary skill in the art may be aware that the units and algorithm steps described in the embodiments disclosed in this specification can be implemented by a combination of electronic hardware, computer software, and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In several embodiments provided in this application, when any function is implemented in a form of a software functional unit and sold or used as an independent product, the function may be stored on a computer-readable storage medium. Based on such an understanding, the technical solutions of this application essentially, or the part contributing to the conventional technology, or some of the technical solutions may be implemented in a form of a software product. The computer software product is stored in a storage medium and includes several instructions for instructing an electronic device (which may be a personal computer, a server, or a network device) to perform all or some of the steps of the methods described in embodiments of this application. The foregoing storage medium includes any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of embodiments of this application. Any variation or replacement readily figured out by a person skilled in the art within the technical scope disclosed in this application shall fall within the protection scope in embodiments of this application. Therefore, the protection scope of this application shall be subject to the protection scope of the claims.

Claims

1. A protection prompt method, applied to an electronic device, wherein the electronic device comprises a first sensor, a second sensor, and a third sensor, and the method comprises:

   obtaining an ultraviolet light intensity captured by the first sensor, a first temperature captured by the second sensor, and a second temperature captured by the third sensor;
   obtaining an ultraviolet radiation amount based on the ultraviolet light intensity;
   obtaining a core body temperature of a user based on the ultraviolet light intensity, the first temperature, and the second temperature; and
   providing a protection prompt for the user based on the ultraviolet radiation amount and the core body temperature.

2. The method according to claim 1, wherein the obtaining the ultraviolet radiation amount based on the ultraviolet light intensity comprises:
   performing cumulative summation on the ultraviolet light intensity to obtain the ultraviolet radiation amount.

3. The method according to claim 1 or 2, wherein the obtaining the core body temperature of the user based on the ultraviolet light intensity, the first temperature, and the second temperature comprises:

   if the ultraviolet light intensity is less than a first threshold, obtaining the core body temperature of the user based on the first temperature and the second temperature; or
   if the ultraviolet light intensity is greater than or equal to the first threshold, obtaining the core body temperature of the user based on the ultraviolet light intensity, the first temperature, and the second temperature.

4. The method according to claim 3, wherein the obtaining the core body temperature of the user based on the first temperature and the second temperature comprises:
   calculating the first temperature and the second temperature by using a multiple linear regression model, to obtain the core body temperature.

5. The method according to claim 3, wherein the obtaining the core body temperature of the user based on the ultraviolet light intensity, the first temperature, and the second temperature comprises:
   calculating the ultraviolet light intensity, the first temperature, and the second temperature by using an XGBoost model, to obtain the core body temperature.

6. The method according to any one of claims 1 to 5, wherein the providing an ultraviolet protection prompt and/or a temperature protection prompt for the user based on the ultraviolet radiation amount and the core body temperature comprises:

   providing the ultraviolet protection prompt for the user based on the ultraviolet radiation amount; and
   providing the temperature protection prompt for the user based on the core body temperature.

7. The method according to claim 6, wherein the providing the ultraviolet protection prompt for the user based on the ultraviolet radiation amount comprises:

   if the ultraviolet radiation amount is less than or equal to a second threshold, skipping providing the ultraviolet protection prompt; or
   if the ultraviolet radiation amount is greater than the second threshold, providing the ultraviolet protection prompt for the user based on the ultraviolet radiation amount.

8. The method according to claim 6, wherein the providing the temperature protection prompt for the user based on the core body temperature comprises:

   if the core body temperature is greater than or equal to a third threshold and less than or equal to a fourth threshold, skipping providing the temperature protection prompt;
   if the core body temperature is less than the third threshold, providing a prompt of paying attention to hypothermia protection for the user based on the core body temperature; or
   if the core temperature is greater than the fourth threshold, providing a prompt of paying attention to heatstroke prevention for the user based on the core body temperature.

9. The method according to any one of claims 1 to 8, wherein the second threshold is a personalized threshold corresponding to a physiological parameter of the user.

10. The method according to any one of claims 1 to 8, wherein before the obtaining the ultraviolet light intensity captured by the first sensor, the first temperature captured by the second sensor, and the second temperature captured by the third sensor, the method further comprises:
obtaining the second threshold in response to a change operation performed by the user on a target control used to display the second threshold.

11. The method according to any one of claims 1 to 10, wherein the first temperature is an ambient temperature, and the second temperature is a skin temperature of the user.

12. The method according to any one of claims 1 to 11, wherein the electronic device comprises smart glasses.

13. The method according to claim 12, wherein a manner of providing the ultraviolet protection prompt and/or the temperature protection prompt for the user comprises one or any combination of the following options: automatically changing a color of a lens, displaying prompt content on the lens with a display function, playing the prompt content through a speaker, and providing the ultraviolet protection prompt and/or the temperature protection prompt through another electronic device.

14. The method according to claim 13, wherein the smart glasses comprise an electrochromic lens.

15. The method according to claim 14, the automatically changing a color of a lens comprises:
adjusting an applied voltage of an electrochromic material in the lens based on the ultraviolet light intensity.

16. The method according to any one of claims 1 to 15, wherein the obtaining the ultraviolet light intensity captured by the first sensor comprises:

obtaining the ultraviolet light intensity captured by the first sensor; and
adjusting an invocation frequency of the first sensor based on the ultraviolet light intensity.

17. The method according to claim 12, wherein the first sensor and the second sensor are located in positions that are in a temple of the smart glasses and that are close to a front panel.

18. The method according to claim 12, wherein the first sensor and the second sensor are located on a front panel of a lens frame of the smart glasses.

19. The method according to claim 18, wherein the first sensor and the second sensor are located in positions that are close to a temple and that are in the front panel of the lens frame.

20. The method according to claim 18, wherein the first sensor and the second sensor are located in middle positions of the front panel of the lens frame.

21. The method according to any one of claims 17 to 20, wherein the second sensor is located outside the smart glasses, and the third sensor is located inside the temple of the smart glasses.

22. The method according to any one of claims 1 to 11, wherein the electronic device comprises a smart watch or a smart headset.

23. An electronic device, comprising a first sensor, a second sensor, a third sensor, a processor, and a memory, wherein the memory is configured to store a computer program, the computer program comprises program instructions, and when the processor runs the program instructions, the electronic device is enabled to perform the steps of the method according to any one of claims 1 to 22.

24. A computer-readable storage medium, wherein the computer-readable storage medium stores a computer program, the computer program comprises program instructions, and when the program requests to be run by a computer, the computer is enabled to perform the method according to any one of claims 1 to 22.

Electronic device 100

Antenna 1

Antenna 2

| Mobile communication module<br>2G/3G/4G/5G [150] | Wireless communication module<br>BT/WLAN/GNSS/NFC/IR/FM [160] |

Speaker
[170A]

Receiver
[170B]

Audio
module
[170]

Microphone
[170C]

Headset
jack [170D]

Displays 1 to N [194]

Cameras 1 to N [193]

Indicator [192]

Motor [191]

Button [190]

Internal memory [121]

SIM card interfaces 1
to N [195]

External memory
interface [120]

Processor
[110]

Sensor module [180]

Pressure sensor
[180A]

Gyro sensor [180B]

Barometric
pressure sensor
[180C]

Magnetic sensor
[180D]

Acceleration sensor
[180E]

Distance sensor
[180F]

Optical proximity
sensor [180G]

Fingerprint sensor
[180H]

Temperature sensor
[180J]

Touch sensor
[180K]

Ambient light
sensor [180L]

Bone conduction
sensor [180M]

USB port [130]

Charging
input

Charging
management
module
[140]

Power
management
module [141]

Battery [142]

FIG. 1

| Application layer | Camera | Calendar | Maps | WLAN | Music | Messaging |
|---|---|---|---|---|---|---|
| | Gallery | Phone | Navigation | Bluetooth | Videos | ... |

| Application framework layer | Window manager | Content provider | Phone manager | Resource manager |
|---|---|---|---|---|
| | Notification manager | View system | ... | |

| System library | Surface manager | Three-dimensional graphics processing library | Android runtime |
|---|---|---|---|
| | Two-dimensional graphics engine | Media library | ... | |

| Kernel layer | Display driver | Camera driver | |
|---|---|---|---|
| | Audio driver | Sensor driver | ... |

FIG. 2

Temple
22

Third sensor
241

First sensor 251

Second
sensor 252

Speaker 243

Processor &
Bluetooth 242

Main PCB 24

FPC flexible flat cable 26

Hinge 23

Front panel 21

Secondary
PCB 25

20

FIG. 3

Ultraviolet radiation
personalized settings

To provide you with a refined
ultraviolet radiation reminder
function, a personalized
reminder is generated for you
based on your skin condition:

Please start the camera and
provide your face photo and
upper arm photo

$\Rightarrow$ $\sim$
TO
FIG. 4B

FIG. 4A

CONT.
FROM
FIG. 4A
~

Ultraviolet radiation
personalized settings

Analyzing a parameter...

~
TO
FIG. 4C

FIG. 4B

Ultraviolet radiation personalized settings

A personalized reminder parameter generated for you is

CONT.
FROM
FIG. 4B
~

| Ultraviolet index | Risk level | Radiation reminder duration (minute) |
|---|---|---|
| 0 to 2 | Weak | - |
| 3 to 5 | Medium | - |
| 6 to 7 | Strong | 35 |
| 8 to 10 | Very strong | 25 |
| ≥11 | Super strong | 12 |

~
TO
FIG. 4D

FIG. 4C

CONT.
FROM
FIG. 4C
~

Ultraviolet radiation
personalized settings

You can manually adjust
reminder duration

| Ultraviolet index | Risk level | Radiation reminder duration (minute) |
|---|---|---|
| 0 to 2 | Weak | - |
| 3 to 5 | Medium | - |
| 6 to 7 | Strong | 35 |
| 8 to 10 | Very strong | 25 |
| ≥11 | Super strong | 12 |

FIG. 4D

FIG. 5

FIG. 6

Reminder
Your face is currently receiving UV light with an index of 6, having a risk of sunburn. It is recommended that your activity time in this environment is no more than 30 minutes, wear a sun hat, and apply a sunscreen

FIG. 7

Warning!
You have been active in an
environment with an ultraviolet
index of 10 for 8 minutes, with a
skin temperature of 36.8°C and a
core body temperature of 37.3°C.
There is a high risk of sunburn and
heatstroke. It is recommended that
you return to a cool place
immediately and take sun
protection measures

FIG. 8

Ultraviolet radiation

xiaoyu
June 8, 2022
Sunny | 35°C | Southeast wind 2

Ultraviolet index

10
6
2

6:00    9:00    12:00    15:00    18:00

Excessively high
High
Medium
Low

3 hour
4 hour
4 hour
13 hour

● Excessively high
● High
● Medium
● Low

FIG. 9

34

Ultraviolet radiation

| | xiaoyu | June 8, 2022 |
| | | Sunny ┊ 35°C ┊ Southeast wind 2 |

Amount of received ultraviolet radiation (J/m$^2$)

600

400

200

6:00      9:00      12:00      15:00      18:00

**Ultraviolet radiation monitoring:**
You have received a total of 2340 J/m$^2$ ultraviolet radiation today and have been active for 2.5 hours in a strong radiation environment with an ultraviolet index greater than 6. Strong ultraviolet exposure is harmful to your skin. It is recommended that you wear a sun hat and apply an SPF 15 and PA+ or higher sunscreen

FIG. 10

FIG. 11

FIG. 12

Obtain an ultraviolet light intensity captured by a first sensor, a first temperature captured by a second sensor, and a second temperature captured by a third sensor ⟋ 302

Obtain an ultraviolet radiation amount based on the ultraviolet light intensity ⟋ 304

Obtain a core body temperature of a user based on the ultraviolet light intensity, the first temperature, and the second temperature ⟋ 306

Provide a protection prompt for the user based on the ultraviolet radiation amount and the core body temperature ⟋ 308

FIG. 13

Adjust an invocation frequency of a first sensor based on an ultraviolet light intensity ⟋ 3042

Obtain radiation time under different ultraviolet light intensities based on the ultraviolet light intensity and the invocation frequency ⟋ 3044

Perform cumulative summation on the ultraviolet light intensity based on the ultraviolet light intensity and the radiation time, to obtain an ultraviolet radiation amount ⟋ 3046

FIG. 14

Determine whether an
ultraviolet light intensity is greater than a
first threshold — 3062

Yes

No

Obtain a core body temperature of a user based on a first
temperature and a second temperature — 3064

Obtain the core body temperature of the user based on the
ultraviolet light intensity, the first temperature, and the second
temperature — 3066

FIG. 15

Provide an ultraviolet protection prompt for a user based on an
ultraviolet radiation amount — 3082

Provide a temperature protection prompt for the user based on a
core body temperature — 3084

FIG. 16

Determine whether an
ultraviolet radiation amount is greater than a second
threshold — a2

No

Yes

Provide an ultraviolet protection prompt for a user based on the
ultraviolet radiation amount — a4

Skip providing the ultraviolet protection prompt for the user — a6

FIG. 17

Determine
whether a core body temperature is greater than a
fourth threshold — b2

No          Yes

Provide a prompt of paying attention to heatstroke prevention for a user
based on the core body temperature — b4

Determine
whether the core body temperature is less than a
third threshold — b6

No

Yes

Provide an ultraviolet protection prompt for the user based on an
ultraviolet radiation amount — b8

The
procedure
ends

FIG. 18

| Obtain a target voltage value based on an ultraviolet light intensity | c2 |

↓

| Adjust an applied voltage of an electrochromic material in a lens based on the target voltage value | c4 |

**FIG. 19**

| Generate prompt content in a text format based on an ultraviolet radiation intensity and/or a core body temperature | d2 |

↓

| Display the prompt content in the text format on a screen of a lens | d4 |

**FIG. 20**

| Generate prompt content in a voice format based on an ultraviolet radiation intensity and/or a core body temperature | e2 |

↓

| Play the prompt content in the voice format through a speaker | e4 |

**FIG. 21**

400

Electronic device

Transceiver unit — 401

Processing unit — 402

FIG. 22

## INTERNATIONAL SEARCH REPORT

| International application No. |
|---|
| **PCT/CN2023/136187** |

| A. | CLASSIFICATION OF SUBJECT MATTER |
|---|---|

G08B21/24(2006.01)i

According to International Patent Classification (IPC) or to both national classification and IPC

| B. | FIELDS SEARCHED |
|---|---|

Minimum documentation searched (classification system followed by classification symbols)

IPC:G08B A61B

Documentation searched other than minimum documentation to the extent that such documents are included in the fields searched

Electronic data base consulted during the international search (name of data base and, where practicable, search terms used)

CNABS, CNTXT: 紫外光, 紫外线, 环境, 皮肤, 温度, 核心, 体温, 防护, 提示, 提醒; VEN, USTXT, EPTXT, WOTXT, ISI: ultraviolet light, ultraviolet radiation, UV, environment, skin, temperature, core, shield, protection, prompt, remind

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | TW 201705902 A (COMPAL ELECTRONICS INC.) 16 February 2017 (2017-02-16) description, paragraphs 11-27, and figures 1-5 | 1-24 |
| Y | CN 113164069 A (ONIO AS) 23 July 2021 (2021-07-23) description, paragraphs 131-154 and 178 | 1-24 |
| Y | CN 103876720 A (DALIAN CHEERING TECHNOLOGY CO., LTD.) 25 June 2014 (2014-06-25) description, paragraphs 27-46, and figures 1-4 | 1-24 |
| Y | CN 110381816 A (ONIO AS) 25 October 2019 (2019-10-25) description, paragraphs 114-138 and 158 | 1-24 |
| A | CN 1885863 A (HANGZHOU BIRD SOFTWARE CO., LTD.) 27 December 2006 (2006-12-27) entire document | 1-24 |
| A | WO 2021016622 A1 (TRUE WEARABLES INC.) 28 January 2021 (2021-01-28) entire document | 1-24 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 February 2024** | **23 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/136187**

| Patent document cited in search report | | | Publication date (day/month/year) | Patent family member(s) | | | Publication date (day/month/year) |
|---|---|---|---|---|---|---|---|
| TW | 201705902 | A | 16 February 2017 | US | 2017042486 | A1 | 16 February 2017 |
| | | | | TWI | 622379 | B | 01 May 2018 |
| CN | 113164069 | A | 23 July 2021 | EP | 3860445 | A1 | 11 August 2021 |
| | | | | EP | 3860445 | B1 | 19 July 2023 |
| | | | | KR | 20210069673 | A | 11 June 2021 |
| | | | | JP | 2022504350 | A | 13 January 2022 |
| | | | | JP | 7340601 | B2 | 07 September 2023 |
| | | | | US | 2022000368 | A1 | 06 January 2022 |
| | | | | WO | 2020071924 | A1 | 09 April 2020 |
| | | | | NO | 201801285 | A | 06 April 2020 |
| | | | | GB | 2580015 | A | 15 July 2020 |
| | | | | GB | 2580015 | B | 12 May 2021 |
| | | | | IN | 202117019933 | A | 21 January 2022 |
| CN | 103876720 | A | 25 June 2014 | None | | | |
| CN | 110381816 | A | 25 October 2019 | US | 2019343397 | A1 | 14 November 2019 |
| | | | | US | 11179041 | B2 | 23 November 2021 |
| | | | | KR | 20190132381 | A | 27 November 2019 |
| | | | | KR | 102532409 | B1 | 15 May 2023 |
| | | | | EP | 3606414 | A1 | 12 February 2020 |
| | | | | EP | 3606414 | B1 | 27 January 2021 |
| | | | | WO | 2018186748 | A1 | 11 October 2018 |
| | | | | DK | 3606414 | T3 | 26 April 2021 |
| | | | | JP | 2020515847 | A | 28 May 2020 |
| | | | | JP | 7125951 | B2 | 25 August 2022 |
| | | | | NO | 201700555 | A | 05 October 2018 |
| | | | | IN | 201917043945 | A | 10 January 2020 |
| | | | | CN | 110381816 | B | 05 August 2022 |
| CN | 1885863 | A | 27 December 2006 | None | | | |
| WO | 2021016622 | A1 | 28 January 2021 | JP | 2022541647 | A | 26 September 2022 |
| | | | | GB | 202202408 | D0 | 06 April 2022 |
| | | | | GB | 2601090 | A | 18 May 2022 |
| | | | | US | 2021022676 | A1 | 28 January 2021 |
| | | | | US | 2022202362 | A9 | 30 June 2022 |

Form PCT/ISA/210 (patent family annex) (July 2022)

**REFERENCES CITED IN THE DESCRIPTION**

**Patent documents cited in the description**

- CN 202211556409